(19) Europäisches Patentamt
European Patent Office
Office européen des brevets

(11) Publication number: 0 119 623 B1

(12) # EUROPEAN PATENT SPECIFICATION

(45) Date of publication of patent specification: **08.01.92** (51) Int. Cl.$^{5}$: **G01N 33/53**

(21) Application number: **84103003.4**

(22) Date of filing: **19.03.84**

(54) **Multilayer analytical element for non-isotopic assay.**

(30) Priority: **17.03.83 JP 45060/83**

(43) Date of publication of application:
**26.09.84 Bulletin 84/39**

(45) Publication of the grant of the patent:
**08.01.92 Bulletin 92/02**

(84) Designated Contracting States:
**DE GB**

(56) References cited:
**EP-A- 0 013 156**
**EP-A- 0 066 648**
**EP-A- 0 069 281**
**EP-A- 0 097 952**

(73) Proprietor: **FUJI PHOTO FILM CO., LTD.**
**210 Nakanuma Minamiashigara-shi**
**Kanagawa-ken, 250-01(JP)**

(72) Inventor: **Yasuda, Yukio**
**c/o Fuji Photo Film Co.Ltd., 3-11-46, Senzui**
**Asaka-shi, Saitama(JP)**
Inventor: **Masuda, Nobuhito**
**c/o Fuji Photo Film Co.Ltd., 3-11-46, Senzui**
**Asaka-shi, Saitama(JP)**

(74) Representative: **Kraus, Walter, Dr. et al**
**Patentanwälte Kraus, Weisert & Partner**
**Thomas-Wimmer-Ring 15**
**W-8000 München 22(DE)**

**EP 0 119 623 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a multilayer analytical element for non-isotopic assay of an analyte contained in a liquid sample. More particularly, this invention relates to a multilayer analytical element appropriately employable for determination of the concentration of a substance (analyte) which plays important roles in bio-chemical process or of various substances originating from living bodies or organisms, using a protein specifically bindable to the analyte in the presence of a non-isotopically labeled analyte or analogue thereof through a competitive binding reaction. This invention further relates to a slide enclosing the multilayer analytical element and a method for non-isotopic assay employing said multilayer analytical element.

In a field of clinical tests, there are known a variety of methods where several reagent solutions are added to a liquid sample and the concentration of a specific component (analyte) in the liquid sample is determined. In these methods, the reaction reagents are necessarily weighed with high accuracy and thoroughly mixed so as to enhance the reproducibility of the determination and, in some occasions, precise separation of the resulting precipitates from the supernatants through centrifugation is required. Thus, these conventional methods inherently require highly trained skills.

Accordingly, certain improved apparatus in which the required procedures are all automated have been proposed so as to replace the above-mentioned conventional method. These automated apparatus are capable of providing high reproducibility without such high skills and are convenient for determination of a large number of liquid samples. Nevertheless, these have serious disadvantages such as high expense. In order to obviate the disadvantages of such automated apparatus, multilayer analytical elements in which reaction reagents are in advance incorporated therein have been proposed as a simple analytical means in which the supply of the reagent solutions is substantially not required, whereby no trained skills are required. These multilayer analytical elements are advantageous in that they are inexpensive as compared with the automated apparatus. However, although various improvements have been proposed with respect to the multilayer analytical elements utilizing chemical reactions or enzymatic reactions, it is still difficult to assay trace components or components having high structural specificity.

For the analysis of such components using reagent solutions, there is known a method utilizing an immunological reaction, that is, an immunoassay. However, only certain numbers of proposals have been made until now with respect to a multilayer analytical element in the form of a film containing the reagents for the immunological reaction. Further, it is noted that most of the known multilayer analytical elements find difficulty in obtaining satisfactory analytical results in the case that they are employed for assay involving an immunological reaction, namely immunoassay. This is because the immunological reaction inherently has specific problems.

For obtaining satisfactory analytical results in the immunoassay, it is very important to introduce a minimum amount of a liquid sample into the multilayer analytical element within a relatively short period, as well as to hold the so introduced liquid sample within the element for a sufficient period to carry out the desired antigen-antibody reaction.

From the above-mentioned viewpoints, the present inventors have studied the previously proposed multilayer analytical elements such as those described in Japanese Patent Publication No. 53(1978)-21677 corresponding to U.S. Patent 3,992,158; Japanese Patent Provisional Publication No. 51(1976)-40191 corresponding to U.S. Patent 4,042,335; Japanese Patent Provisional Publication No. 56(1981)-24576; Japanese Patent Provisional Publication No. 55(1980)-90859 corresponding to U.S. Patent 4,258,001; and Japanese Patent Provisional Publication No. 53(1978)-131089 corresponding to U.S. Patent 4,144,306.

As a result of these studies, the present inventors have noted that the previously known multilayer analytical elements subjected to their study all are unsatisfactory in the capacity for holding the liquid sample within the element. Accordingly, these multilayer analytical elements show disadvantageous problems in the accuracy, sensitivity and rapidity.

The present inventors already invented a multilayer analytical element comprising an integrated detection member which comprises a reaction layer for carrying out a reaction between a specific component (analyte) and a protein specifically bindable to the analyte, and a detection layer receiving a product showing a signal modulated in response to the concentration of the analyte. This invention was applied for U.S. Patent under Serial Number 446 110 on Dec. 2, 1982 , and disclosed in Japanese Patent Provisional Publication No. 57(1982)-200862 and EP 0 066 648 A1. The multilayer analytical element according to the above-mentioned invention has successively solved the problem with respect to holding the liquid sample within the element. In this multilayer analytical element, a competitive antigen-antibody reaction is carried out in the reaction layer. When the reaction is complete, the resulting bound labeled complex (B) remains in the reaction layer, while the unreacted (free) labeled substance (F) diffuses into the detection layer placed under the reaction layer, in which the free substance (F) is photometrically detected.

The above-mentioned analytical element allows a sufficient amount of a liquid sample to participate in the competitive antigen-antibody reaction as compared with the conventional multilayer analytical elements, and thus assures high analytical reproducibility at high sensitivity. Accordingly, this element was considered to solve the difficulty in providing a rapid and simple analytical means, inherently attached to the immunoassay in the dry analysis system, by accomplising separation of the bound labeled complex (B) from the free labeled substance (F), namely B/F separation, similtaneously with progress of the antigen-antibody reaction in the multilayer analytical element.

The necesssity of the B/F separation have brought about various difficulties in the immunoassay. In the first place, B/F separation to a satisfactory level is as such rather difficult, and requires a lengthy period. In the second place, the B/F separation procedure is difficultly incorporated into an automated continuous analytical process. Because of such difficulty in the B/F separation, not only high skill is required in the analytical procedure, but also the reliability of the analytical data is reduced. For coping with these problems, Japanese Patent Provisional Publications Nos. 53(1978)-79024 and 53(1978)-38619 disclose analytical processes in which the fluorescence of the labeled substance is determined in the reaction mixture without B/F separation, that is, no separation between the bound labeled complex (B) and the free labeled substance (F) is involved. However, these analytical processes are only applicable to a specific analytical system in which the fluorescence of the bound labeled complex (B) is stronger or weaker than that of the free labeled substance (F), and are not widely applicable to a normal or ordinary antigen-antibody reaction. Other disadvantageous features of these analytical processes comprising no B/F separation resides in that fluorescence of the background is also detected, whereby increasing noise and lowering the S/N (signal/noise) ratio. Also disadvantageous is a need of trained high skills in, for instance, measuring the reagents involved in the analysis. Moreover, although such measuring procedure can be incorporated into the automated apparatus to simplify the total procedure, such apparatus becomes highly expensive.

The present inventors discovered that the defective features of the conventional arts can be effectively overcome by a multilayer analytical element comprising specifically arranged members to accomplish the B/F separation within the analytical element. This invention has provided a multilayer analytical element for dry analysis comprising (a) a fibrous or non-fibrous porous spreading sheet containing a certain amount of a fluorescent substance-labeled analyte or analogue thereof, (b) a porous sharing sheet, and (c) a porous reaction sheet containing a certain amount of a protein fixed therein, the protein being specifically bindable to the analyte, which are laminated in this order, and has been disclosed in Japanese Patent Application No. 57(1982)-114359 (corresponding to U. S. Serial No. 508,518 and EPA 83106271).

## SUMMARY OF THE INVENTION

A primary object of the present invention is to provide a multilayer analytical element showing reduced noises, accordingly, showing a high S/N ratio.

Another object of the invention is to provide a multilayer analytical element for non-isotopic assay in which the B/F separation is done within the element and the signal of the bound labeled complex (B) can be directly determined.

There is provided by the present invention a multilayer analytical element for non-isotopic assay of an analyte contained in an acqueous liquid sample comprising a light-transmissive water-impermeable support (131, 331) and sheets containing a non-isotopically labeled analyte substance or analogue thereof and a protein being specifically bindable to the analyte, which is characterized in that said sheets comprise:

a sheet (132, 332) provided on the support, which comprises a continuous binder matrix containing the non-isotopically labeled analyte substance or analogue thereof in a uniform amount per unit area;

a porous reaction sheet (122, 322) provided on the continuous binder matrix sheet, which contains a predetermined amount of the protein fixed therein; and

a porous sharing sheet (121, 321) provided on the porous reaction sheet.

A slide for non-isotopic assay

encloses the above-mentioned multilayer analytical element within a frame having on a surface of the porous sharing sheet side an opening for introduction of the liquid sample and on a surface of the support side an opening for enabling photometric measurement.

A method for non-isotopic assay

employs the above-mentioned multilayer analytical element or the slide and is characterized in that the substantial separation of the bound non-isotopically labeled complex (B) from the free non-isotopically labeled analyte substance or analogue thereof (F) is accomplished by transfer of the free non- isotopically labeled analyte substance or analogue thereof (F) into a space different from the porous reaction sheet

upon the competitive binding reaction.

In the present invention, the term "analyte substance" means the same substance as an analyte to be analyzed. For instance, if the analyte in the liquid sample is thyroxine, a predetermined amount of a non-isotopically labeled analyte substance, that is, a non-isotopically labeled thyroxine, is subjected to a competitive antigen-antibody reaction in the presence of the unknown amount of the analyte (thyroxine). An analogue of the analyte substance can be also subjected to the competitive antigen-antibody reaction in the same manner to perform the non-isotopic assay such as a non-isotopic immunoassay. Accordingly, the term "analyte substance" used hereinunder means to include an analyte substance and an analogue thereof, unless otherwise specified.

Figure 1 is a vertical section view showing the essential layer structure of a multilayer analytical element for non-isotopic assay according to the present invention.

Figure 2 is a perspective view of the multilayer analytical element shown in Figure 1.

Figure 3 is a vertical section view of an embodiment of a slide for non-isotopic assay enclosing a multilayer analytical element within a frame according to the present invention, in which Figure 3a is a vertical section view of the slide; Figure 3b is a top plane view of the slide (upper member of frame); Figure 3c is a vertical section view of a upper member of the frame taken along line I-I in Figure 3b; Figure 3d is a bottom plane view of the frame and Figure 3e is a vertical section view of a lower member of the frame taken along line I-I in Figure 3d.

Figure 4 is a calibration curve indicating a relationship between a relative fluorescence strength and $T_4$ content in a serum obtained in the use of a slide for quantitative analysis of $T_4$ described in Example 4.

In the figures, the numerals mean the following:

121, 321: porous sharing sheet,
122, 322: porous reaction sheet,
131, 331: light-transmissive water-impermeable support,
132, 332: sheet comprising a continuous binder containing a non-isotopically labeled substance,
333 : timing sheet,
351 : upper frame,
352 : lower frame,
X : opening for introduction of aqueous liquid sample,
Y : opening for photometric measurement, and
I-I : lines along which the section views are taken.

The analytical element of the present invention is employable for non-isotopic assay of an analyte contained in an aqueous liquid sample, in which the assay comprises:

a stage of subjecting the analyte, a predetermined amount of a protein specifically bindable to the analyte and a predetermined amount of a non-isotopically labeled analyte substance or analogue thereof, to competitive binding reaction;

a stage of substantially separating the resulting bound non-isotopically labeled complex (B) of the protein from the free non-isotopically labeled analyte substance or analogue thereof (F); and

a stage of determining the signal strength of said non-isotopically labeled complex (B).

In the multilayer analytical element, the above-mentioned substantial separation of the bound non-isotopically labeled complex (B) of the protein from the free non-isotopically labeled analyte substance (F), namely B/F separation, is accomplished by the presence of the porous sharing sheet (b), which shall be occasionally referred to simply as "sharing sheet", provided therein.

The sharing sheet functions for sharing the free non-isotopically labeled substance (F) into other space than the space of the porous reaction sheet containing a predetermined amount of the protein fixed therein, in which the protein is specifically bindable to the analyte.

In the present invention, the term "sharing" means "introducing the free non-isotopically labeled substance (F) into other space than the space of the reaction sheet". As is be described hereinafter, the multiple layers of the analytical element of the invention can be so designed that the free non-isotopically labeled substance (F) can be shared into the sharing sheet in an amount as much as possible, whereby most of the free non-isotopically labeled substance (F) is introduced into a space other than the space of the reaction sheet, while the reaction sheet contains substantially only the bound non-isotopically labeled complex (B). Therefore, the B/F separation is sufficiently accomplished, and the strength of a signal of the bound non-isotopically labeled complex (B) can be independently determined.

Accordingly, the term "separation" used in the present invention means separation of the bound non-isotopically labeled complex (B) from the free non-isotopically labeled substance (F) resulting from:

the first stage in which an applied liquid sample is distributed within the multilayer analytical element keeping an equal amount per unit area and the analyte in the liquid sample is uniformly distributed within

the analytical element, as well as the non-isotopically labeled analyte substance contained in the continuous binder sheet is uniformly distributed within the analytical element through dissolution in the medium of the liquid sample;

and,

the second stage in which the analyte and the non-isotopically labeled analyte substance react competitively to the beforehand fixed specifically bindable protein in the reaction sheet, resulting in production of a bound non-isotopically labeled complex (B) within the reaction sheet.

The above-mentioned first and second stages are not thought to occur independently from each other, that is, these phenomena mentioned in both stages are thought to take place under microscopically mixed conditions immediately after applying (e.g., dropping or spotting) a liquid sample onto the analytical element, and after lapse of a certain period (incubation period) the B/F separation is substantially attained.

In the above-mentioned case, the non-isotopically labeled analyte substance contained in the continuous binder sheet is dissolved in the medium of the liquid sample immediately after contact therebetween, to diffuse in the direction to the reaction sheet (b) and the sharing sheet (c), in which the introduced analyte and the labeled analyte substance undergo the competitive binding reaction to the specifically bindable protein beforehand contained and fixed in the reaction sheet (b). Otherwise the dissolution of the labeled substance into the medium of the liquid sample from the continuous binder sheet can be delayed so that the diffusion of the labeled substance into the reaction sheet (b) and the sharing sheet (c) can be delayed. In this case, the analyte in the liquid sample is first subjected to the binding reaction with the specifically bindable protein fixed beforehand in the reaction sheet (b), and subsequently the competitive binding reaction among the four components, namely, the labeled substance having diffused therein, a free analyte in the liquid sample, a complex of a portion of the analyte in the liquid sample and the protein, and a free specifically bindable protein proceeds in the reaction sheet (b), to produce a bound non-isotopically labeled complex (B). This procedure is termed "labeled substance-delayed addition method", and is effective for enhancement of the analytical sensitivity. Accordingly, the delayed addition method is preferably employed in the present invention.

The reaction sheet provides a space for performing the competitive binding reaction between the non-isotopically labeled analyte substance and the analyte to the specifically bindable protein fixed therein. As a result of the competitive bindable reaction, an optical signal is produced in the reaction sheet from the photometrically detectable bound non-isotopically labeled complex (B). The complex (B) is thus produced under substantial separation from the free (unreacted) non-isotopically labeled substance (F) distributed into the sharing sheet, which is a space different from the reaction sheet. The term "substantial separation" includes a case in which the bound non-isotopically labeled complex (B) is located only in the reaction sheet and most of the free non-isotopically labeled substance (F) is located in a space such as the sharing sheet other than the reaction sheet upon sharing, while a portion of the labeled substance (F) still remains in the reaction sheet. Accordingly, the signal strength measured on the reaction sheet includes not only the signal strength of the bound complex (B), but also that of the free non-isotopically labeled substance (F) located in the reaction sheet. Thus measured signal strength value is plotted against a predetermined calibration curve to determine the amount of the analyte.

If the analytical element is so designed as to increase the sharing ratio for space other than the reaction sheet, the influence on the signal strength given by the free non-isotopically labeled substance (F) can be reduced to a negligible level. Accordingly, the so designed analytical element is a preferred embodiment of the present invention.

The sharing ratio of the free non-isotopically labeled substance (F) between the reaction sheet and other space than the reaction sheet depends upon nature of the material, thickness and thickness ratio of the reaction sheet and one or more sheets providing the space other than the reaction sheet, affinity of the analyte and the non-isotopically labeled analyte substance to the material constituting each sheet, and the like. For instance, the sharing ratio can be adjusted by such a simple manner that each sheet is prepared to have a controlled liquid holding capacity which means a volume of a liquid which can be held within the sheet.

In the above, the liquid holding capacity is determined by multiplying the volume of the sheet by the void ratio. In the present invention, the above-mentioned substantial separation can be accomplished by adjusting the liquid holding capacity ratio bewteen the sharing sheet and the reaction sheet to a value in the range of from 1 : 3 to 5 : 1. If the liquid holding capacity of the sharing sheet increases relative to that of the reaction sheet, the amount shared into the sharing sheet increases. Thus, the above-mentioned liquid holding capacity ratio preferably is in the range of from 1 : 1 to 3 : 1. If the liquid holding capacity is less than 1 : 3, the B/F separation is not effectively accomplished and the S/N ratio is kept at a low level. On the other hand, various difficulties reside in preparation of a multilayer analytical element having a liquid holding

capacity more than 5 : 1.

If the material of the sharing sheet is the same as that of the reaction sheet, the liquid holding capacity ratio is generally determined according to the thickness of each sheet. However, even if the ratio in thickness between the sharing sheet and the reaction sheet is equivalent to 1 : 1, the sharing degree can be increased to provide a greater amount of the free non-isotopically labeled substance (F) into the sharing sheet by, for instance, increasing the liquid holding capacity per thickness of the sharing sheet as compared with that of the reaction sheet, or incorporating a protein having a lower binding constant which is different from the protein fixed in the reaction sheet, or a compound having affinity of the labeled substance, into the sharing sheet.

In the case that the non-isotopically labeled subtance (F) is a fluorescent substance-labeled substance, a bound fluorescent complex of the beforehand fixed specifically bindable protein and the fluorescent substance-labeled substance (B) is produced in the reaction sheet, while the free fluorescent substance-labeled substance (F) is shared between the reaction sheet and the sharing sheet. A radiation is then applied to the multilayer analytical element from the transparent support side to excite the bound fluorescent complex (B), and a light emitted by the bound fluorescent complex (B) is detected along a direction different from the direction of the regular reflection Thus, the signal strength of the bound fluorescent complex (B) is measured.

The fluorescent substance-labeled analyte substance can be prepared in a conventional manner by combining the analyte substance with a fluorescent substance directly or indirectly through a bifunctional compound such as an amino acid, a dicarboxylic acid, or a diamino compound. Such combining processes have been heretofore reported with respect to a variety of analytes, and are well known to those skilled in the art. For instance, the details are given in CLINICAL TEST TECHNIQUE, Vol. 4, Immunoserological Test, edited by Tadashi Kawai (Igaku Shoin, Japan, 1977), pp. 97-102; Japanese Patent Provisional Publications Nos. 53(1978)-79024 and 53(1978)-142522; Biochem. Biophys. Res. Commun. 74, 538(1977); and Clin. Chim. Acta, 83, 161(1978).

The fluorescent substance-labeled analyte substance can be prepared in any of the conventional processes, provided that the analyte substance still retains the reactivity to the protein fixed in the reaction sheet. The indirect combination between the analyte substance and the fluorescent substance through the bifunctional compound is frequently utilized to assure the fixed protein of satisfactory reactivity. In the present invention, such indirectly fluorescent substance combined substances are termed "fluorescent substance-labeled analyte analogue". Further known in the art is a technique in which a variety of appropriate functional groups can be introduced into the fluorescent substance-labeled substance at sites not participating in the binding reaction so as to effectively carry out the competition with the analyte in the binding reaction to the protein fixed in the reaction sheet, or to improve the solubility. In the present invention, the substance modified as above is also termed "fluorescent substance-labeled analyte analogue".

Representative examples of the fluorescent substance include fluorescein isothiocyanate (FITC), methylrhodamine, umbelliferone, N-methyl-2-anilino-6-naphthalenesulfonate, and ε-[5-(dimethylamino)naphthalene-1-sulfonyl]lysine.

The fluorescent substance-labeled analyte substance is bound to the specifically bindable protein fixed in the reaction sheet in the course of the competitive binding reaction to produce a bound fluorescent substance-labeled complex (B). In the conventional fluoroimmunoassay, fluorescence strength of the bound fluorescent substance-labeled complex (B) or the free fluorescent substance-labeled substance (F) is measured.

However, it is known that in a specific case the quantitative assay of an analyte can be carried out through measurement of change of the fluorescence strength between before and after the antigen-antibody reaction. For instance, it is known that the fluorescence strength of a bound fluorescent substance-labeled complex (B) increases after an antigen-antibody reaction in a certain system (e.g., system described in Japanese Patent Provisional Publication No. 53(1978)-79024). Also known is a reverse system in which the fluorescence strength decreases after an antigen-antibody reaction (e.g., system described in Japanese Patent Provisional Publication No. 53(1978)-38619 corresponding to U.S. Patent 4,150,949 and OLS(DE) 27 16 276).

Either of the above-mentioned reaction systems can be applied to the present invention. Naturally, a system in which no substantial change of the fluorescence strength between before and after an antigen-antibody reaction is observed can be applied to the present invention. The last-mentioned system is viewed to be not utilizable in a fluoroimmunoassay without B/F separation.

In the case that the non-isotopic label is a fluorescent label, the multilayer analytical element of the present invention may further comprise a light shielding substance to improve the accuracy of the

fluorometric measurement. For this purpose, a fibrous porous material mentioned hereinafter as materials of the sharing sheet can be incorporated after being colored with a dye having absorption band in a wavelength region of the exciting radiation and/or radiating fluorescence (e.g., reactive dye, and vat dye). Otherwise, pigment particles of white or black or colored pigments can be incorporated into the sharing sheet. Moreover, the above-mentioned dye can be fixed to the non-fibrous particles mentioned hereinafter as materials of the sharing sheet. Also employable is the non-fibrous particles containing the colored pigment. By the incorporation of the light shielding substance in the manner as above, the multilayer analytical element of the present invention is given a light shielding function and advantageously employed in the photometric measurement.

Representative examples of the light shielding substance include white fine powders such as $TiO_2$ fine powder, ZnO fine powder and $BaSO_4$ fine powder, and white or pale glossy metal fine powder such as aluminum fine powder.

If the light shielding substance is incorporated into the analytical element as above, the fluorescence emitted by the serum components, of which wavelength is shorter than wavelength of the fluorescence to be measured, is absorbed thereby to reduce the blank value derived from the serum component.

In the case that the non-isotopic label is an enzyme, it is required that the enzyme-labeled substance (F) is bound to the specifically bindable protein contained in the reaction sheet to produce an enzyme-labeled complex (B), the enzymatic activity of which is different from that of the free enzyme-labeled substance (F). An enzymatic reaction proceeds simultaneously with the binding reaction if a substrate of the enzyme-labeled substance (F) is present in the reaction sheet. The change of the amount of the substrate caused by the enzymatic reaction is proportional to the ratio between the amount of the enzyme-labeled complex (B) and the free enzyme-labeled substance (F). For instance, a theophylline derivative labeled with glucose-6-phosphate dehydrogenase (G-6-PDH) can be employed as the enzyme-labeled substance in the quantitative determination of theophylline in a liquid sample, as described in Japanese Patent Provisional Publication No. 53(1978)-130692.

One embodiment of the multilayer analytical element of the present invention is an analytical element comprising a continuous binder sheet containing the enzyme-labeled substance therein, a porous reaction sheet containing a certain amount of a bindable protein fixed therein, and a porous sharing sheet containing light-shielding particles. The enzymatic reaction caused by the enzyme-labeled substance proceeds in the presence of glucose-6-phosphate (G6P: substrate of the enzyme) and nicotinamide-adenine dinucleotide (NAD: coenzyme) which is introduced by one of the following procedures: a procedure involving beforehand incorporation thereof into the continuous binder sheet, a procedure of introducing these substances together with the liquid sample in the form of a liquid mixture, or a procedure of placing a buffer solution of the enzyme substrate and coenzyme onto the analytical element after spotting the liquid sample on the element. The change of the amount of the substrate affected by the produced substance can be detected by reflection photometry at 340 nm or fluorometry at 450 nm (wavelength of excitation: 348 nm). The enzymatic activity originating from the G6P-labeled substance greatly decreases when the labeled substance reacts with the bindable protein to produce an enzyme-labeled complex (B). In more detail, if theophylline is present in the liquid sample in a small amount, most of the enzyme-labeled theophylline reacts with the bindable protein and accordingly is present in the form of a complex (B). Thus, little enzymatic activity is observed. On the other hand, if theophylline is present in the liquid sample in a great amount, only a small portion of the enzyme-labeled theophylline reacts with the bindable protein and accordingly most of the enzyme labeled substance (i.e., theophylline) is present in the free form. Thus, the enzymatic activity is substantially maintained.

The free enzyme-labeled substance (F) is shared between the porous sharing sheet and the reaction sheet. Nevertheless, the change of amount of the substrate caused by the enzymatic reaction of the free enzyme-labeled substance (F) located in the reaction sheet can be photometrically measured substantially independently from the change occurring in the sharing sheet, because the product given by the enzymatic reaction in the sharing sheet is photometrically masked by the light-shielding solid particles. Accordingly, the porous sharing sheet containing the light-shielding solid particles is advantageous for enhancing the S/N ratio.

Examples of the light-transmissive water-impermeable support include films of plastic materials such as cellulose esters (e.g., cellulose diacetate, cellulose triacetate, and cellulose acetate propionate), polycarbonate of bisphenol A, polyethylene terephthalate, polystyrene, polymethyl methacrylate, polyvinyl chloride, poly(vinylidene chloride - vinyl chloride), polytetrafluoroethylene, polyethylene, polypropylene, and polyamide (e.g., nylon 6); and a glass plate.

The thickness of the support generally is in the range of from approx. 50 $\mu m$ to approx. 2,000 $\mu m$, preferably from 80 $\mu m$ to 1,000 $\mu m$.

The continuous binder sheet comprising a continuous binder matrix which contains the non-isotopically labeled analyte substance or analogue thereof in a substantially uniform amount per unit area (i.e., the content of the labeled substance per unit area is maintained substantially at a definite level over the sheet) can be prepared by coating on the support a dispersion of the non-isotopic labeled substance and binder in a volatile solvent and then removing the volatile solvent from the coated layer.

The continuous binder sheet swells with absorption of the liquid sample or is gradually dissolved in the liquid sample at the surface upon contact with the liquid supplied through the sharing sheet and the reaction sheet. Simultaneously with this phenomenon, the non-isotopic labeled substance contained in the binder sheet is released into the liquid sample.

Examples of the material of the continuous binder include film-forming hydrophilic polymers such as natural polymers, e.g., gelatin, gelatin derivatives, polysaccharide, carboxymethylcellulose, and hydroxyethylcellulose; and synthetic polymers, e.g., homopolymers and copolymers of monomers such as vinyl alcohol, vinylpyrrolidone, acrylic acid derivatives, methacrylic acid derivatives and sulfostyrene. Also employable is a dispersed particulate polymer latex. The continuous binder can contain a film-hardening agent, a pH buffer, an anionic or nonionic surfactant, a pigment, and a dye.

The non-isotopically labeled substance can be incorporated into the above-mentioned continuous binder by adding a solution of the labeled substance to the coating solution (or dispersion) and coating the resulting mixture on the support. Otherwise, the labeled substance can be incorporated in the form that the labeled substance is supported on a particulate carrier, a high molecular protein (e.g., bovine serum albumin, rabbit serum albumin, and human serum albumin), an ionic or nonionic surfactant, or colloidal silica. Also employable is a method which comprises forming a continuous binder film on the support; immersing the binder film in a labeled-substance solution (or dispersion) to introduce the labeled substance onto or into the the binder film and then drying the binder film.

The rate of dissolution of the labeled substance upon contact with the liquid sample is influenced by molecular size of the labeled substance and strength of interaction between the material of the continuous binder sheet and the labeled substance. Accordingly, the rate of dissolution of the labeled substance can be adjusted by varying the material of the continuous binder sheet or introducing a hydrophilic polyelectrolyte or an ionic surfactant. Otherwise, the dissolution rate of the labeled substance can be adjusted by varying the thickness of the continuous binder sheet.

The porous sharing sheet can be made of a fibrous porous material. Examples of the fibrous porous material include natural fibers such as pulp, cotton, silk, and wool: semisynthetic fibers such as cellulose esters and viscose rayon; synthetic fibers such as polyamides (e.g., nylon 6, nylon 66, and nylon 610), polyesters (e.g., polyethylene terephthalate), and polyolefins (e.g., polypropylene); and fibrous inorganic materials such as glass fibers, colored glass fibers, and asbestos. These materials can be employed in such forms as fabrics (woven or knitted), felts, and non-woven fabrics.

The above-described material can be employed as the sharing sheet after incorporating fine particles therein. These fine particles serve as fillers for filling in part the voids of the fibrous porous material to render the manner of permeation of a liquid anisotropic. Examples of the fine particles employable for these purposes include fine particles of non-fibrous polymers of polysaccharides such as agar, agarose, sepharose, sephadex and dextran, polyacrylamide, cellulose (powder), and latex prepared by polymerization or copolymerization of polymerizable ethylenic polymers. Such non-fibrous fine particles as agar, agarose, and sepharose® are able to increase the liquid holding capacity per thickness of the sheet, whereby enabling enhancement of the sharing ability without increase of the thickness of the sharing sheet. The fine particles can be incorporated into the sharing sheet in an amount of up to approximately 90 % by weight based on the total amount of the so incorporated sheet.

The porous sharing sheet can be made of a non-fibrous isotropically-porous material. Examples of the non-fibrous isotropically-porous material include blushed polymers (generally called "membrane filter"); materials in which micro-porous particles such as diatomaceous earth and micro-crystalline particles (e.g., micro-crystalline cellulose) is dispersed; porous materials containing spherical micro-beads of glass and synthetic polymers under point-to-point contact with binder; and blushed polymers containing fine powder of $TiO_2$, $BaSO_4$, mica substantially uniformly dispersed therein.

As the fine-particles for incorporation into the fibrous porous material or the blushed polymer, optically non-transparent particles such as colored pigments (e.g., particles of water-insoluble inorganic or organic coloring compound), white pigments (e.g., light-reflective $TiO_2$, $BaSO_4$ and mica), and water-insoluble carrier particles having a coloring compound on the surface (e.g., water-insoluble agarose particles combined chemically with a dye residue) are preferred. The S/N ratio of the strength of the signal produced in the reaction sheet can be enhanced by rendering the sharing sheet non-transparent through the incorporation of the non-transparent particles thereinto.

The thickness of the porous sharing sheet generally ranges from approx. 80 μm to approx. 1,000, preferably from approx. 150 μm to approx. 500 μm.

Under the sharing sheet is provided a porous reaction sheet.

The reaction sheet contains a predetermined amount of a specifically bindable protein fixed therein to cause a binding reaction competitively between the analyte and the non-isotopically labeled analyte substance. The material of the reaction sheet is generally selected from the materials given hereinbefore for the sharing sheet.

The specifically bindable protein is a protein capable of participating in the competitive binding reaction between the analyte and the non-isotopically labeled analyte substance. A representative example of the specifically bindable protein is an antibody. The specifically bindable protein is so automatically determined to correspond to the analyte in view of the specific reaction concerned, and is physically (e.g., through adsorption) or chemically (e.g., through covalent or noncovalent bonding) fixed in a material constituting the reaction sheet in a conventional manner.

As the methods for incorporating the specifically bindable protein into the reaction sheet under fixing, the following methods are known:

(1) a method in which the protein is bound to a fibrous porous material by linking directly the immunoglobulin to the fibrous material through the amino groups or carboxyl groups by a physical process (e.g., by adsorption) or a chemical process (e.g., by covalent bonding) or by linking indirectly through linking components by these processes; and

(2) a method employable in the case of incorporating fine particles in which the protein is peviously provided to the fine particles (e.g., agarose: otherwise, the fine particles hereinbefore given as examples of fine particles to be incorporated into the sharing sheet can be employed for this purpose) through covalent bonding or adsorption or through linking components capable of specifically binding Fc fragments (e.g., secondary antibody, protein A).

Details of the fixing methods are given in Japanese Patent Provisional Publication No. 47(1972)-18597.

The protein fixed in the reaction sheet generally has a binding constant in the range of $10^7$ to $10^9$ l/mol, and is automatically determined to correspond to the analyte in view of the binding reaction to the analyte.

The protein fixed in the reaction sheet shows its specifically bindable property to an analyte or a non-isotopically labeled analyte substance upon contact with a liquid sample. The thickness of the reaction sheet generally ranges from approx. 100 μm to approx. 1,000 μm, preferably from approx. 200 μm to approx. 500 μm.

The multilayer analytical element of the present invention has, as the requisite members, a support; (a) a sheet of the continuous binder containing the non-isotopically labeled substance; (b) a porous reaction sheet; and (c) a porous sharing sheet. However, one or more subsidiary sheets can be provided to the multilayer analytical element of the invention. For instance, one or more optional layers can be provided between the support, (a) sheet, (b) sheet, and (c) sheet. More specifically, a timing sheet comprising a continuous binder for controlling the rate of the dissolution of the non-isotopic labeled substance can be provided between the (a) sheet and the (b) sheet. A porous layer serving as a subsidiary spreading sheet for assisting the spreading of the applied liquid sample can be superposed over the sharing sheet. An anti-curling layer in the form of a continuous binder sheet such as a gelatin-coated layer can be provided on the surface of the support (the surface not facing the laminated structure) for keeping the support from curling. Otherwise, a material having a selective absorption region can be incorporated into the above-mentioned continuous binder material for reducing the noises introduced in the photometrical measurement process.

One or more sheets of the multilayer analytical element of the present invention may contain the following known reagents required or preferred in the non-isotopic assay or other reagents.

(1) Blocker agent

In certain cases, an analyte to be assayed is under engagement with polymer substances such as albumin and globulin in the serum so that the desired reaction (e.g., specific binding reaction) hardly takes place. For instance, thyroxine ($T_4$) is generally bound to albumin. In other cases, a fluorescent substance-labeled analyte substance is engaged with serum components so that the specifically bindable ability in an antigen-antibody reaction is reduced. For releasing or reducing such undesirable engagement between the analyte or fluorescent substance-labeled substance and the serum component, a reagent generally called "blocker agent" may be incorporated.

Representative examples of the blocker agent include 8-anilino-1-naphthalenesulfonic acid (ANS), thimerosal, and salicylic acid.

(2) Buffer agent

Various buffer agents may be incorporated to provide a pH range suitable for allowing the maximum function of the blocker agent (pH 6 - 11); a pH range suitable for the competitive binding reaction (pH 7 - 10); and others.

Examples of the buffer agent employable in the invention include known buffer agents described in CHEMICAL HANDBOOK, FUNDAMENTAL EDITION edited by the Chemical Society of Japan (Maruzen, Tokyo, 1966), pp. 1312 - 1320; Biochemistry, 5(2), 467 - 477(1966) "Hydrogen Ion Buffers for Biological Research" by N. E. Good et al.; Analytical Chemistry, 104, 300 - 310(1980) "Hydrogen Ion Buffers for Biological Research" by W. J. Ferguson et al.; and others. More concretely, the buffer agent may be glycine, borate, phosphate or diethyl-barbiturate.

(3) Preserving agent

The preserving agent is incorporated for keeping the specifically bindable protein from rotting, preventing moisture absorption, controlling the hygroscopic degree. As an example, sodium aside can be mentioned.

(4) Dissolving or Spreading assistant agent

Water soluble solids such as sucrose, amino acids and glycerol, or various surfactants such as anionic surfactants (e.g., sodium alkylbenzenesulfonate) and nonionic surfactants (e.g., polyethylene glycol) may be incorporated for assisting dissolution of the above-mentioned reagents and further for assisting spreading of these reagents quickly and uniformly.

(5) Other substances

Bovine serum albumin (BSA), rabbit serum albumin (RSA), human serum albumin (HSA), gelatin, and others are preferably incorporated for preventing pseudo reactions to assist smooth and stable progress of the antigen-antibody reactions, whereby improving the accuracy and repeatability of the measurements.

Among the above-mentioned reagents and other substances, the blocker agent is preferably incorporated into the subsidiary spreading sheet, while the buffer agent, preserving agent and spreading agent may be incorporated into the sharing and/or the reaction sheet according to the analytical object.

The analyte which can be assayed by the use of the multilayer analytical element of the invention is a high molecular or low molecular substance present in a bio-chemical component-containing liquid or a body fluid such as blood, plasma, serum, spinal fluid, saliva, urine, etc.

Representative examples of the analyte are as follows.

(1) Simple proteins: for instance, protamine, albumin, and globulins (e.g., α-globulin, β-globulin, particularly immunoglobulin, IgG, IgA, IgE, IgM, IgD), screloproteins (structural proteins such as collagen, elastin, and actin)

(2) Conjugated proteins: for instance, mucoprotein, chromoprotein, lipoprotein, nucleoprotein, glycoprotein, and phosphoprotein

(3) Enzymes, Complements, and Peptide hormones

(4) Antigenic low molecular weight substances: antigenic low molecular weight substances have a molecular weight of generally from 100 to 2,000, ordinarily 125 to 1,000. Examples include the following drugs, agricultural chemicals, small peptides, amino acids, low molecular weight hormones, and metabolites thereof;

(a) Drugs: alkaloids (morphine, codeine, kinine, digoxine), 5- or 6-membered lactams (barbiturate ), amino-alkylbenzenes (amphetamine, epinephrine, catecholamine), benzo-heterocyclic compounds (oxazepam, chlorpromazine), purines (theophylline, caffeine), vitamins (A, B complex, C, D, E ), prostaglandins, antibiotics (penicillin, tetracycline, cephalosporin), aminoglycosides (gentamycine, kanamycine), other drugs (methadone, meprobamate, lidocaine, griseofulvin), and metabolites of these drugs (b) Agricultural chemicals: halogenated biphenyls, phosphoric acid esters, thiophosphate, and metabolites thereof

(c) Small peptides, amino acids, low molecular weight hormones, triiodothyronine, thyroxine ($T_4$), encepharine, bradykinine, angiotensine I and II, and metabolites thereof

The multilayer analytical element of the present invention is further described hereunder by referring to the attached drawings.

Figure 1 is a section view showing the essential layer structure of a multilayer analytical element of the invention. On the light-transmissive water-permeable support 131, a sheet of continuous binder containing a non-isotopically labeled substance 132, a porous reaction sheet 122, and a porous sharing sheet 121 are laminated in order. Figure 2 is a perspective view of the multilayer analytical element illustrated in Figure 1.

Figure 3 is a vertical section view of an embodiment of a slide for non-isotopic assay

in which the numeral 351 indicates a upper frame having an opening X for introduction of a liquid sample and the numeral 352 indicates a lower frame having an opening Y for photometric measurement. The upper frame and the lower frame are combined by an adhesive, ultrasonic processing or heat-processing to give an integrated structure. Within the frame, a light-transmissive water-impermeable support 331, a sheet of continuous binder containing the labeled substance 332, a timing sheet 333, and a porous sharing sheet 321 are laminated in this order. The plane views and section views of the uppor frame 351 and the lower frame 352 are given in Figures 3b, 3c, 3d, and 3e.

As described above, it is apparent that the present invention is different from the prior arts and has new characteristic features.

More in detail, the present invention is different from the art disclosed in the aforementioned Japanese Patent Provisional Publication No. 57(1982)-200862 having a detection sheet and a reaction sheet which comprises a fibrous porous medium containing a specifically bindable protein in the following features: in the present invention, (1) a product giving a photometrically detectable signal is produced in the reaction sheet and retained therein when the signal is measured, and (2) the position of the labeled substance is defined within the sheet of continuous binder (continuous binder sheet) provided between the support and the reaction sheet.

The present invention is different from the multilayer analytical element disclosed in the aforementioned Japanese Patent Application No. 57(1982)-114359 comprising (a) a fibrous or non-fibrous porous spreading sheet containing a predetermined amount of a fluorescent substance-labeled analyte substance or analogue thereof, (b) a porous sharing sheet, and (c) a porous reaction sheet containing a specifically bindable protein, in the following features: in the present invention, (1) the labeled substance is located on the side opposite the sharing sheet via the reaction sheet, and (2) the sheet containing the labeled substance is not a porous sheet but a continuous binder sheet.

The present invention relating to the multilayer analytical element containing a non-isotopically labeled substance is further described by the following examples. However, the following examples should not be construed to restrict the present invention. Examples 1 and 2 are concerned with the multilayer analytical element containing a fluorescent substance-labeled substance as the non-isotopically labeled substance. As the non-isotopically labeled substance, an enzyme-labeled substance or a substance having a chemiluminescent group can be utilized in the conventional manner.

Example 1

(1) Preparation of Fluorescein-labeled Thyroxine (FITC-$T_4$)

FITC-$T_4$ having the following structure was prepared by a series of the procedrues i), ii) and iii) given below.

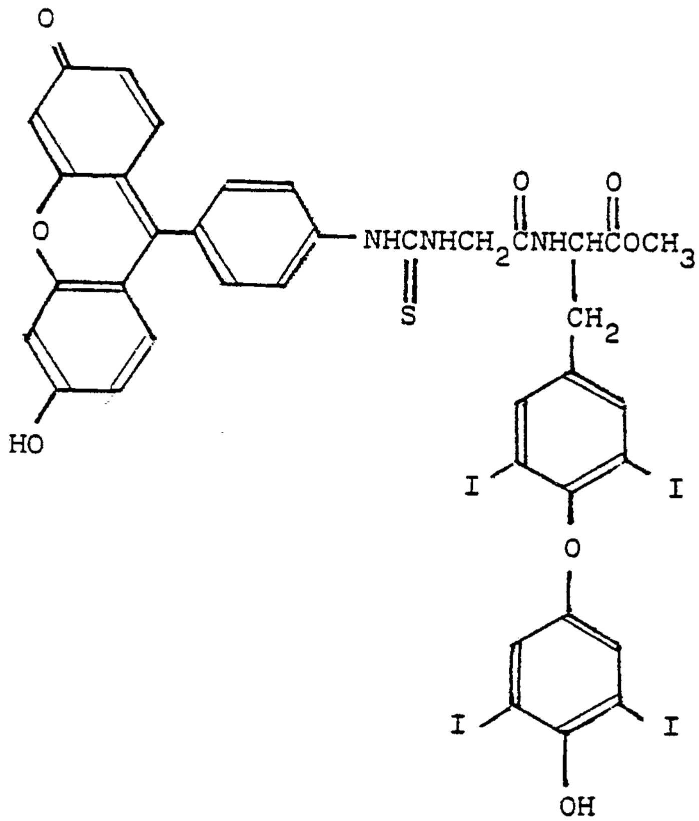

i) Preparation of t-butoxycarbonylglycyl thyroxine methyl ester (Boc-Gly-$T_4$-$OCH_3$)

In 5 ml. of tetrahydrofuran (THF), 329 mg. (1.21 mmol) of t-butoxycarbonylglycine succinyl ester (Boc-Gly-OSu) was dissolved. Independently, 1.0 g. (1.21 mmol) of thyroxine methyl ester hydrochloride is dissolved in 15 ml. of THF, and to this solution was added 170 µl. (1.21 mmol) of triethylamine ($Et_3N$) in 3 ml. of THF.

To the Boc-Gly-OSu solution was added under ice-cooling the thyroxine methyl ester solution. The resulting mixture was allowed to stand to reach room temperature, and then stirred for 1.5 hrs. The reaction liquid was allowed to stand overnight, and to this were added 25 ml. of distilled water and 50 ml. of ethyl acetate for extraction with the ethyl acetate. The extraction with 25 ml. of ethyl acetate was repeated three times. The ethyl acetate extracts were combined and dried over anhydrous magnesium sulfate. The dry extract was concentrated in a rotary evaporator and subjected to gel chromatography using a column filled with Sephadex® LH-20. In this procedure, a mixture of acetone and methanol in a volume ratio of 4 : 1 was employed as the solvent. While confirming by TLC, the fractions containing the desired product were collected and concentrated under reduced pressure to obtain a crystalline product. There was thus obtained 950 mg. (yield 82.5%) of the desired product.

ii) Removal of BOC

950 mg. (1 mmol) of Boc-Gly-$T_4$-$OCH_3$ prepared in the above i) was dissolved in 10 ml. of trifluoroacetic acid. The resulting mixture was stirred for 10 min. under ice-cooling, and subjected to evaporation under reduced pressure at a temperature of below 40°C. The residue was subjected to gel chromatography under the same conditions as in the above i) to collect fractions containing the desired product. Upon crystallization, there was obtained 700 mg. (yield 82.5 %) of the desired product.

iii) Preparation of Fluorescein isothiocyanic acid glycylthyroxine methyl ester (FITC-Gly-$T_4$-$OCH_3$)

320 mg. (0.33 mmol) of Gly-$T_4$-$OCH_3$ trifluoroacetic acid salt obtained in the above ii) and 47 µl. (0.33 mmol) of triethylamine were dissolved in 5 ml. of methanol. The resulting solution was added to 130 mg.

(0.33 mmol) of FITC in 45 ml. of methanol. After reaction was carried out for 24 hrs. at room temperature, the solvent was removed under reduced pressure at a temperature below 40 °C. The residue was subjected to gel chromatography under the same conditions as in the above i) to collect fractions containing the desired product. Upon crystallization, there was obtained 290 mg. (yield 70 %) of an orange colored crystalline product.

The so obtained product was identified by Elementary anlysis, NMR spectrum and Mass spectrum.

FITC-$T_4$ prepared as above was a labeled substance which was able to participate in a competitive antigen-antibody reaction in conjunction with thyroxine ($T_4$). In this antigen-antibody reaction, it was noted that a bound FITC-labeled antigen-antibody complex (B) showed a fluorescence strength approximately twice as much as that shown by a free non-isotopically labeled substance.

(2) Preparation of Multilayer Analytical Element

i) Continuous binder sheet containing fluorescent substance-labeled substance

On a surface of a transparent polyethylene terephthalate film (thickness 180 µm) was applied glow discharge treatment. On the treated surface, a solution containing 5 g. of lime-treated gelatin, 0.05 g. of gelatin hardener, 25 g. of N-vinylpyrrolidone - acrylic acid copolymer (90 : 10, molar ratio), 260 µg. of the labeled substance synthesized in the above (1), and 0.05 g. of sodium dodecylbenzenesulfonate in 100 ml. of water was coated and dried to give a continuous binder sheet of thickness of 1.9 µm. (dry basis).

ii) Reaction sheet

A glass fiber filter paper (Glass Fiber Filter Paper GA-100, produced by Toyo Filter Paper Co., Ltd., Japan) was dispersed in water and broken. Subsequently, the dispersion was processed over filters to give a dispersion containing glass fibers passing through Tyler standard sieve of "12 mesh" but not passing through the "32 mesh" sieve. Independently, an anti-$T_4$ rabbit serum was fixed to surfaces of agarose particles in a conventional manner and 0.45 ml. of 1:1 dispersion of the so treated agarose particles was mixed with 30 ml. of the above-mentioned aqueous glass fiber dispersion (solid content 8 mg.). The resulting mixture was filtered over a circular micro-filter having diameter of 47 mm (prepared from a micro-porous membrane filter available from Fuji Photo Film Co., Ltd., Japa). The material collected over the filter was freeze-dried in the composite form to form a porous reaction sheet containing a specifically bindable protein.

iii) Porous sharing sheet

30 ml. of the glass fiber dispersion (solid content 8 mg) as prepared in the above was combined with 0.7 ml. of 1:1 dispersion of agarose particles colored with orange dye (Mikacion Brill. Orange GS, available from Nippon Kayaku Co., Ltd., Japan). The resulting mixture was filtered over the reaction sheet on the microfilter having diameter of 47 mm prepared in the above ii). The material collected over the reaction sheet was freeze-dried in the composite form to form a colored sharing sheet. The micro-filter was then removed to collect the composite of the reaction sheet and the sharing sheet.

(3) Preparation of analytical slide

Within a set of the upper frame 351 and the lower frame 352, both having been prepared by polyethylene containing black pigment, the continuous binder sheet (diameter 12 mm) containing fluorescent substance-labeled substance 330 and the reaction sheet (diameter 12 mm) having thereon the orange-colored sharing sheet 320 were enclosed to form a laminated structure. Thus, an analytical slide was prepared.

(4) Measurement on Thyroxyine ($T_4$)

On the sharing sheet of the multilayer analytical elements was dropped 80 µl. of a mixture of 40 µl. of thyroxine($T_4$)-containing serum for calibration purpose (prepared by treating a serum with active carbon to obtain a $T_4$ free serum and adding a predetermined amount of thyroxine to the serum) of differnet known concentration, 20 µl. of 0.3-M glycine-NaCl-NaOH buffer, and 20 µl. of 0.1-M aqueous blocker agent (sodium dodecylsulfonate) solution. The multilayer analytical element was incubated at 25 °C for 30 min.

After the incubation was complete, reflective fluorometric measurement was done on the side of the reaction sheet. The reflective fluorometric measurement was carried out at exciting rays of a wavelength 495 nm and a fluoroscence of a wavelength 525 nm using a fluorophotometer 650-10(S) (manufactured by Hitachi, Ltd., Japan).

The calibration curve obtained by the above-measurement is illustrated in Figure 4.

Accordingly, it has been confirmed that $T_4$ present in a serum sample in an amount in the range of 2 - 100 $\mu$g/dl can be determined.

Example 2

(1) Porous sharing sheet

In 30 ml. of the same glass fiber dispersion (solid content 8 mg.) as employed in Example 1 - ii) was dispersed 17 mg. of Pearl Pigment TP-900 (tradename of Teikoku kako Co., Ltd., Japan, consisting mainly of mica having particle size of approx. 50 - 100 $\mu$m). The resulting dispersion was filtered over the same reaction sheet as prepared in Example 1 - ii) and then freeze-dried to form a reaction sheet having a reflective and optically opaque sharing sheet.

(2) Preparation of analytical slide

The same procedure for the preparation of analytical slide as in Example 1 was repeated except that the reaction sheet having orange colored sharing sheet was replaced with the above-mentioned reaction sheet having the white (opaque) sharing sheet.

(3) Measurement on Thyroxyine ($T_4$)

A calibration curve was prepared in the same manner as in Example 1 - (4) using the above analytical slide. The fluorescence strength was 3.77 mV for a serum containing 2 $\mu$g/dl of $T_4$, while 2.49 mV for a serum containing 100 $\mu$g/dl of $T_4$. Thus, it has been confirmed that the flurescence strength increases as much as approx. 2.5 times, as compared with that given in Example 1.

**Claims**

**1.** A multilayer analytical element for non-isotopic assay of an analyte contained in an aqueous liquid sample comprising a light-transmissive water-impermeable support (131, 331) and sheets containing a non-isotopically labeled analyte substance or analogue thereof and a protein being specifically bindable to the analyte, **characterized** in that said sheets comprise:

a sheet (132, 332) provided on the support, which comprises a continuous binder matrix containing the non-isotopically labeled analyte substance or analogue thereof in a uniform amount per unit area;

a porous reaction sheet (122, 322) provided on the continuous binder matrix sheet, which contains a predetermined amount of the protein fixed therein; and

a porous sharing sheet (121, 321) provided on the porous reaction sheet.

**2.** The analytical element as claimed in claim 1,
wherein said non-isotopically labeled analyte substance or analogue thereof is a fluorescent substance-labeled analyte substance or analogue thereof.

**3.** The analytical element as claimed in claim 1,
wherein said non-isotopically labeled analyte substance or analogue thereof is an analyte substance or analogue thereof labeled with an enzyme, the enzymatic activity of which is capable of being modulated by an antigen-antibody reaction.

**4.** The analytical element as claimed in claim 1,
wherein said non-isotopically labeled analyte substance is a non-isotopically labeled antigen or antibody.

**5.** The analytical element as claimed in claim 1,
wherein a ratio of liquid-holding capacity between the porous sharing sheet and the porous reaction

sheet is in the range of 1:3 to 5:1.

6. The analytical element as claimed in claim 1, wherein said porous sharing sheet is made of fibrous porous material.

7. The analytical element as claimed in claim 1, wherein said porous sharing sheet contains a water-insoluble light-shielding material.

8. The analytical element as claims in claim 1, wherein said porous sharing sheet contains water-insoluble light-shielding particles of polysaccharide or a derivative thereof.

**Revendications**

1. Un élément d'analyse multicouche pour l'examen non isotopique d'une substance à analyser contenue dans un échantillon de liquide aqueux comprenant un support imperméable à l'eau transmettant la lumière (131, 331) et des feuilles contenant un analyte ou son analogue marqué par voie non isotopique et une protéine susceptible de se fixer spécifiquement sur l'analyte, caractérisé en ce que lesdites feuilles comprennent :

   une feuille (132, 332) disposée sur le support, qui comprend une matrice de liant continue contenant l'analyte ou son analogue marqué par voie non isotopique en quantité uni forme par unité de surface ;

   une feuille de réaction poreuse (122, 322) disposée sur la feuille de matrice de liant continue, qui contient une quantité prédéterminée de la protéine fixée dans celle-ci ; et

   une feuille poreuse de partage (121, 321) disposée sur la feuille poreuse de réaction.

2. L'élément d'analyse selon la revendication 1, dans lequel ledit analyte ou son analogue marqué par voie non isotopique est un analyte ou son analogue marqué par une substance fluorescente.

3. L'élément d'analyse selon la revendication 1, dans lequel ledit analyte ou son analogue marqué par voie non isotopique est un analyte ou son analogue marqué par une enzyme, dont l'activité enzymatique peut être modulée par une réaction antigène-anticorps.

4. L'élément d'analyse selon la revendication 1, dans lequel ledit analyte marqué par voie non isotopique est un antigène ou un anticorps marqué par voie non isotopique.

5. L'élément d'analyse selon la revendication 1, dans lequel le rapport de la capacité de retenue de liquide de la feuille poreuse de partage à celle de la feuille poreuse de réaction est dans la gamme de 1 : 3 à 5 : 1.

6. L'élément d'analyse selon la revendication 1, dans lequel ladite feuille poreuse de partage est faite d'une matière poreuse fibreuse.

7. L'élément d'analyse selon la revendication 1, dans lequel ladite feuille poreuse de partage contient une matière d'écran à la lumière insoluble dans l'eau.

8. L'élément d'analyse selon la revendication 1, dans lequel ladite feuille poreuse de partage contient des particules d'écran à la lumière insolubles dans l'eau de polysaccharide ou d'un de ses dérivés.

**Patentansprüche**

1. Mehrschichtiges analytisches Element für einen nicht-isotopischen Assay eines Analyten, der in einer wäßrigen flüssigen Probe enthalten ist, umfassend einen lichtdurchlässigen wasserundurchlässigen Träger (131, 331) und Schichten, die eine nicht-isotopisch markierte Analytensubstanz oder ein Analogon davon und ein Protein, das spezifisch an den Analyten binden kann, enthalten, dadurch **gekennzeichnet,** daß die Schichten

   eine Schicht (132, 332) auf einem Träger, umfassend eine kontinuierliche Bindermatrix, die die nicht-isotopisch markierte Analytensubstanz oder ein Analogon davon in einer gleichförmigen Menge

pro Flächeneinheit enthält;
eine poröse Reaktionsschicht (122, 322) auf der kontinuierlichen Bindermatrixschicht, die eine vorbestimmte Proteinmenge in Fixierung daran enthält und
eine poröse Aufteilungsschicht (121, 321) auf der porösen Reaktionsschicht umfassen.

2. Analytisches Element nach Anspruch 1, dadurch **gekennzeichnet,** daß die nicht-isotopisch markierte Analytensubstanz oder das Analogon davon eine fluoreszente Substanz-markierte Analytensubstanz oder ein Analogon davon ist.

3. Analytisches Element nach Anspruch 1, dadurch **gekennzeichnet,** daß die nicht-isotopisch markierte Analytensubstanz oder das Analogon davon eine Analytensubstanz oder ein Analogon davon, das mit einem Enzym markiert ist, ist, wobei die enzymatische Aktivität davon über eine Antigen-Antikörper-Reaktion moduliert werden kann.

4. Analytisches Element nach Anspruch 1, dadurch **gekennzeichnet,** daß die nicht-isotopisch markierte Analytensubstanz ein nicht-isotopisch markiertes Antigen oder Antikörper ist.

5. Analytisches Element nach Anspruch 1, dadurch **gekennzeichnet,** daß das Verhältnis der Flüssigkeitszurückhaltenden Kapazität zwischen der porösen Aufteilungsschicht und der porösen Reaktionsschicht im Bereich von 1:3 bis 5:1 liegt.

6. Analytisches Element nach Anspruch 1, dadurch **gekennzeichnet,** daß die poröse Aufteilungsschicht aus fibrösem porösen Material hergestellt ist.

7. Analytisches Element nach Anspruch 1, dadurch **gekennzeichnet,** daß die poröse Aufteilungsschicht ein wasserunlösliches lichtabschirmendes Material enthält.

8. Analytisches Element nach Anspruch 1, dadurch **gekennzeichnet,** daß die poröse Aufteilungsschicht wasserunlösiche lichtabschirmende Teilchen aus Polysaccharid oder einem Derivat davon enthält.

FIG.1

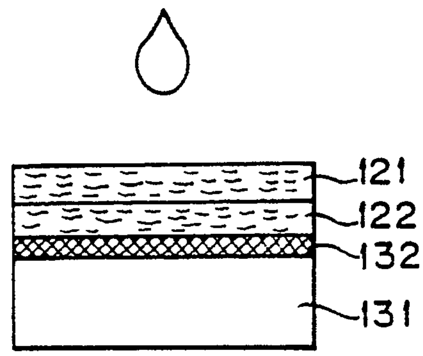


FIG.2

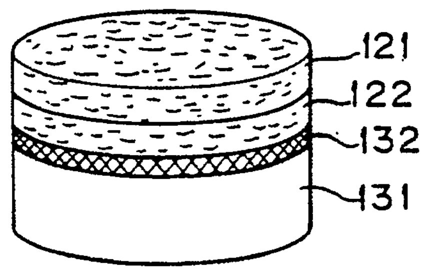


FIG.3a

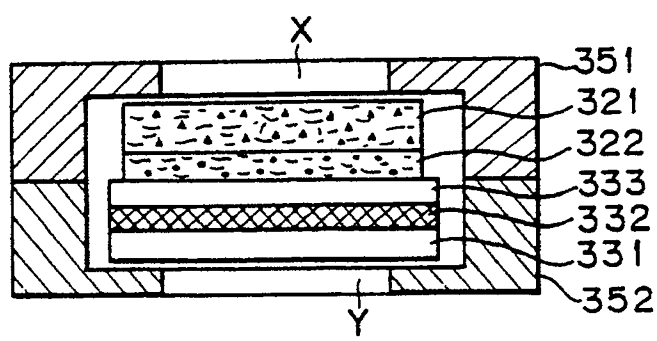


FIG.3b

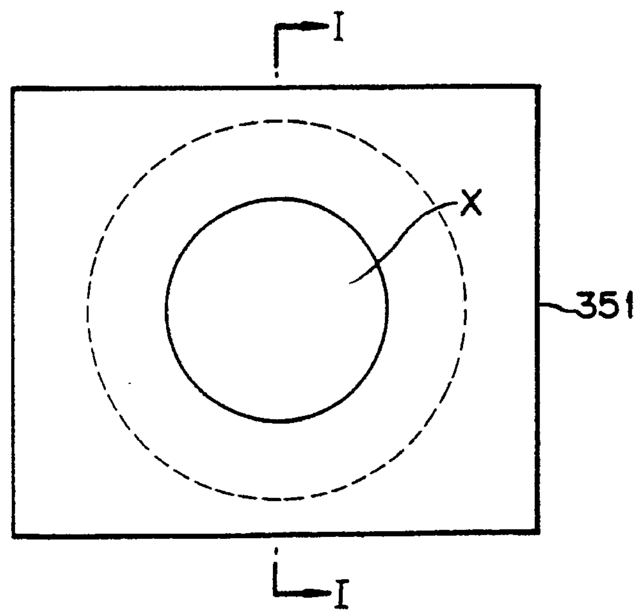

FIG.3c

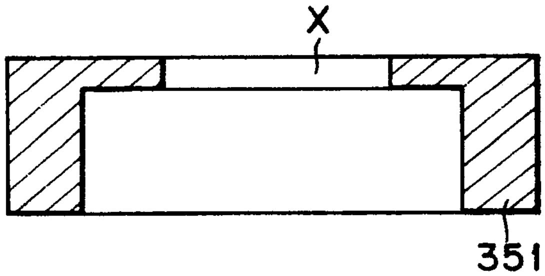


FIG.3d

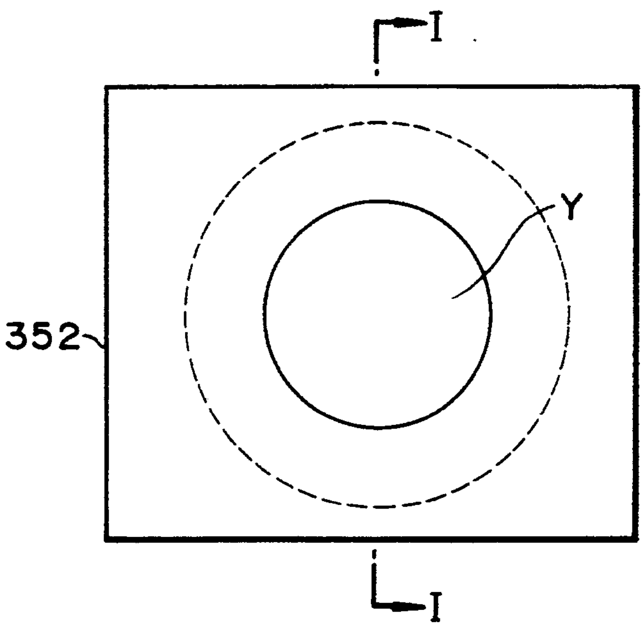


FIG.3e

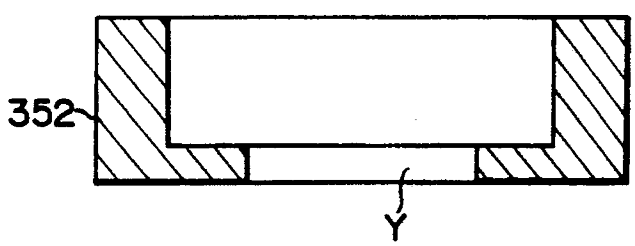

FIG.4

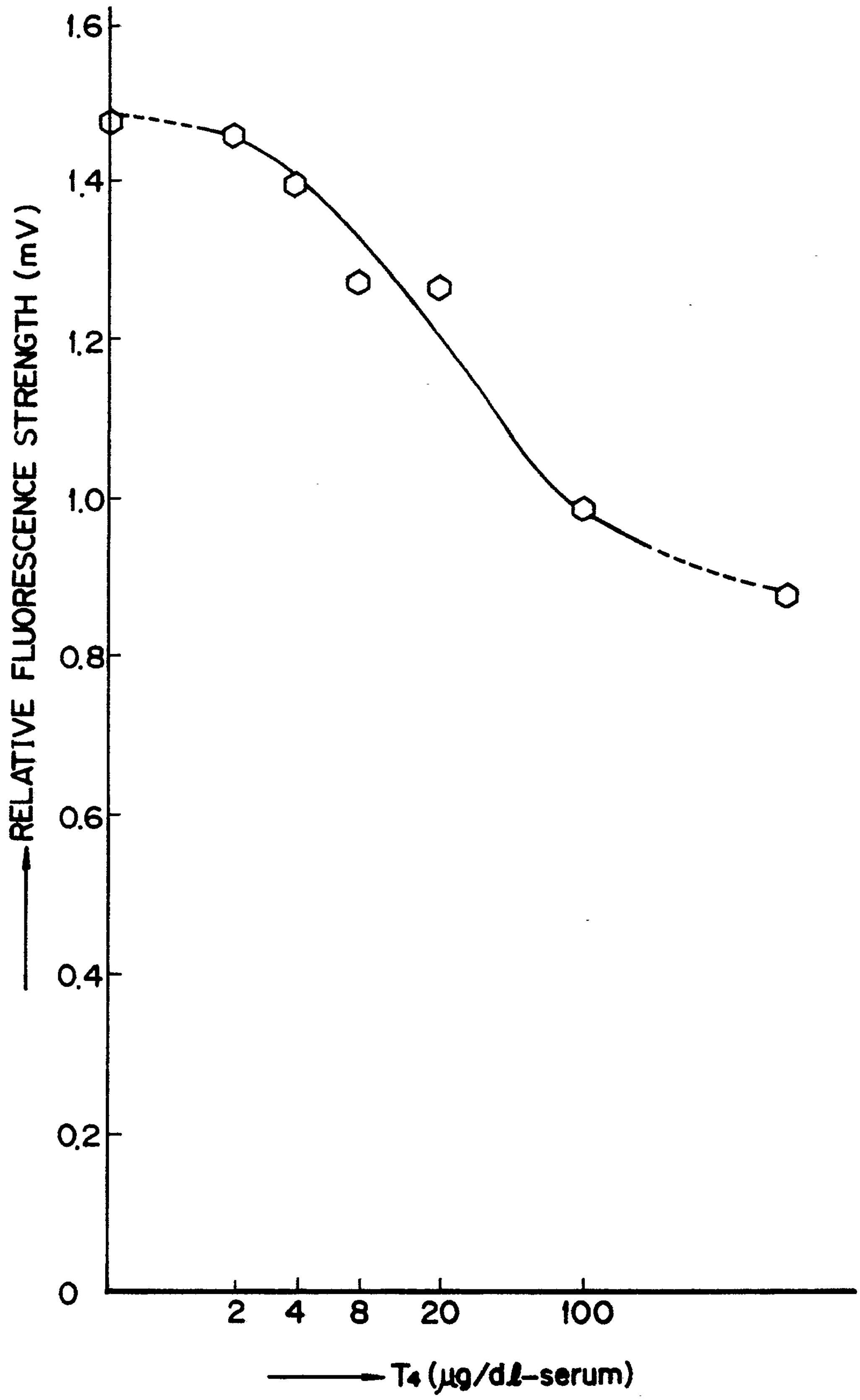
1.6
1.4
1.2
1.0
0.8
0.6
0.4
0.2
0
RELATIVE FLUORESCENCE STRENGTH (mV)
2
4
8
20
100
T4 (μg/dℓ-serum)